# EUROPEAN PATENT APPLICATION

(11) **EP 1 557 184 A1**
(43) Date of publication of application: **27.07.2005**
(21) Application number: 05250230.9
(22) Date of filing: 18.01.2005
(51) Int. Cl.: A61M 1/00, F16K 31/00, F16K 31/56, F16K 37/00, F16K 21/18

(54) **Medical overflow protective device**

(30) Priority: 23.01.2004 US 764020
(71) Applicant: Datex-Ohmeda, Inc., Madison, WI 53718 (US)
(72) Inventor: Hodge, Colin G., Ellicott City, MD 21042 (US); Severns, Matthew L., Gaithersburg, MD 20878 (US)
(74) Representative: Hedley, Nicholas James Matthew

(57) **Abstract**

A protective device (18) that is used in a medical suction system withdrawing liquids from a patient. The device is located upstream of a vacuum regulator (10) and protects that regulator and other downstream components from contacting the liquid being withdrawn from the patient. The protective device has an inlet (24) and an outlet (22) and an actuator mechanism (26) that can move between a contracted position (Fig. 4) where a gas can communicate through the protective device between the inlet and the outlet and an expanded position (Figure 5) where the actuator mechanism occludes both the inlet and the outlet to shut off the vacuum supply to the patient. An electrical circuit having a pair of sensing electrodes (54,55) provides an electrical discharge to trigger a shape-metal alloy wire (58) to move the actuator mechanism from the contracted position to the expanded position when the sensing electrodes detect the presence of a liquid therebetween.

## Description

### Background

The present invention relates to a medical suction system and, more particularly, to an overflow protection device that senses a liquid in a medical suction system to provide protection to components of that system.

It is a common practice in the care of patients, particularly after surgery, to provide a vacuum system that carries out the withdrawal of liquids from the patient. To that end, most hospitals normally have a pipeline supply or source of vacuum that is piped to certain hospital rooms where the suction or withdrawal of liquids is being carried out. Alternatively, there may be an on-site source of vacuum actually within the hospital room itself that provides the necessary vacuum for withdrawal of the liquids.

In either event, there is normally also a vacuum regulator that is present within the treatment room so that the level of vacuum can be regulated by the caregiver to a particular level of vacuum that is desired, and of course, safe, to effectively apply to the particular cavity of a patient for the withdrawal of liquid therefrom.

One of the difficulties, however, in the use, for example, of a central hospital supply of the vacuum is that it is necessary to protect the vacuum regulator, as well as any further pipes, downstream equipment etc. from contamination by the biological liquids that are being removed from the patient. While the various components upstream of the vacuum regulator can be of a type that are readily cleaned or, alternatively, disposable, the downstream components and equipment including the vacuum regulator itself are of a nature that cleaning is very difficult or even not possible should the contaminated liquids actually enter the hospital piping of the central vacuum system.

Accordingly, such vacuum systems normally have some device that senses or somehow is responsive to the presence of a liquid and which device then occludes the vacuum line in some manner that the liquid is prevented from traveling further downstream of that safety device.

As such, therefore, one typical protective device is a collection bottle that collects the liquid from the patient and which has a ball valve and a hydrophobic filter and, while that technology generally operates well and is effective, the actual implementation of the ball valve is large and the device must be oriented in a generally vertical position to be reliable.

Accordingly, it would be advantageous to have a medical suction overflow protective device that is relatively inexpensive, effective, reliable and which provides a good visible indication that it has been activated to occlude the vacuum line. It would also be advantageous for such a protective device to safely contain even that liquid removed from a patient that has entered the protective device to activate the occlusion of the vacuum line so that there is an assurance that such liquid will not leak out of the protective device and contaminate other areas of the hospital.

### Summary of the Invention

Accordingly, the present invention relates to a medical suction overflow protective device that can be used in the vacuum line withdrawing liquid from a patient and provide protection for any components that are downstream of the device including the vacuum regulator. As used herein, the term upstream will refer to the direction of flow of the liquid in the vacuum system, thus, the patient is the ultimate upstream location and the source of vacuum is the ultimate downstream location.

With the protective device of the present invention, there is a housing having an inlet and an outlet and which normally provides a fluid path for the vacuum that draws the liquids from the patient toward the vacuum regulator. Thus, the present protective device is located upstream of the vacuum regulator, preferable adjacent that vacuum regulator and prevents the liquid from the patient from reaching the vacuum regulator. The outlet of the present protective device is thus adapted to be connected to the vacuum regulator and the inlet connected to the patient circuit including medical tubing that eventually leads to the patient via a collection bottle and the like.

An actuator mechanism is contained within the housing and that actuator mechanism has opposed blocking surfaces that are positioned, respectively, adjacent to the inlet and the outlet of the protective device. The actuator mechanism is movable between a contracted position wherein the blocking surfaces are displaced away from the inlet and outlet and an expanded position wherein the blocking surfaces block both the inlet and the outlet of the protective device.

The actuator mechanism is normally biased toward its expanded position with a latch mechanism that retains the actuator mechanism in its contracted position. A pair of sensing electrodes are located along the external surface of the actuator mechanism and detect the presence of a liquid therebetween. When the liquid has been detected between the sensing electrodes, an electrical circuit, preferably battery powered, creates a brief electric discharge through a shape-metal alloy actuator that contracts to release the actuator mechanism from its contracted position so that it expands outwardly to its expanded position and which causes the blocking surfaces to occlude both the inlet and the outlet of the protective device.

With both the inlet and the outlet immediately closed upon the sensing of the liquid withdrawn from a patient, not only is the vacuum regulator downstream of the protective device protected from intrusion of the biologic liquid, but the liquid that has inadvertently entered the protective device itself is also isolated since both the inlet and the outlet are rapidly closed such that any such liquid is effectively trapped within the protective device and can be disposed of in a safe manner without further leakage.

As a further feature of the present protective device, there is a visual indication to the attending personnel that the protective device has been triggered to block the vacuum line and that visible indication can be a brightly colored diaphragm that is initially collapsed when the actuator mechanism is in its contracted position and has not been activated but expands to become very visible through a transparent housing as the actuator mechanism itself expands to its expanded position so as to alert the attending personnel to the fact that the protective device has been triggered and the vacuum line is closed. The overall protective device is also relatively independent with respect to orientation, that is, it functions in almost any orientation.

These and other features and advantages of the present invention will become more readily apparent during the following detailed description taken in conjunction with the drawings herein.

### Brief Description of the Drawings

FIG. 1 is a front view of a conventional vacuum regulator having a medical overflow protective device constructed in accordance with the present invention affixed thereto;
FIG. 2 is a cross-sectional view of the medical overflow protective device of the present invention in its contracted position;
FIG. 3 is a cross-sectional view of the medical overflow protective device of the present invention in its expanded position;
FIG. 4 is a cross-sectional view of the actuator mechanism of the present invention in its contracted position;
FIG. 5 is a cross-sectional view of the actuator mechanism of Fig. 4 in its expanded position; and
FIG. 6 is a schematic view of an electrical circuit that can be used with the present invention.

### Detailed Description of the Invention

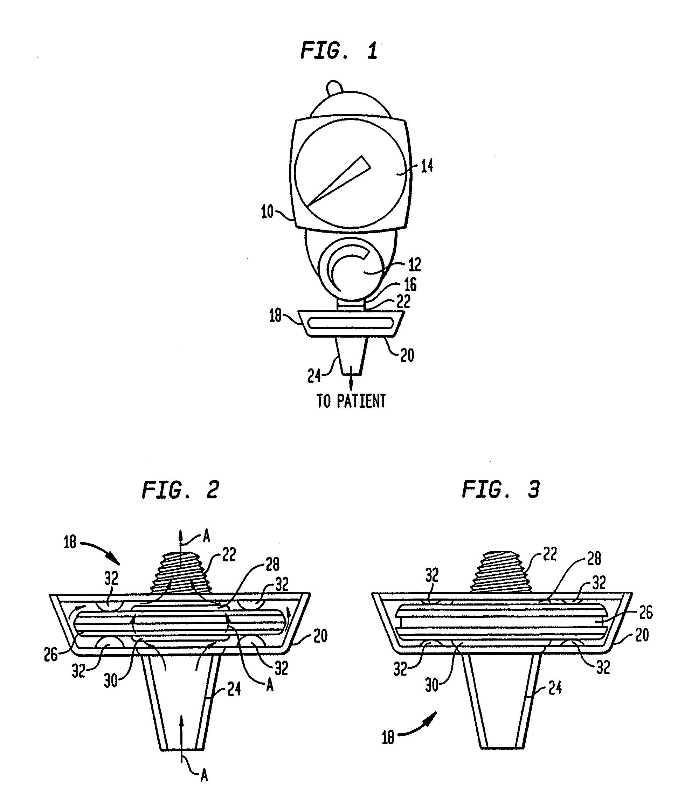

Referring now to Fig. 1, there is shown a front view of a vacuum regulator 10 that can be a conventional construction and which normally includes an external knob 12 that is manipulated by the user to establish the desired level of vacuum upstream of the vacuum regulator 10. There is also normally provided, a gauge 14 that provides a visual indication to that user of the level of vacuum that has been thereby established. The vacuum regulator 10 has an inlet 16 and an outlet (not shown) that is adapted to be connected to the source of vacuum such as the hospital central pipeline of vacuum. By use of the vacuum regulator 10, therefore, there is a vacuum level established at the inlet 16 in accordance with a setting that is established by the user via the external knob 12 and which is ultimately transmitted to a patient by a patient circuit that includes various medical tubing and generally a collection container that retains the fluids drained from the patient.

As also can be seen, there is a protective device 18 that is affixed in fluid communication with the vacuum regulator 10 and the protective device 18 comprises a housing 20 that is preferably constructed of a transparent plastic material and has an outlet 22 and an inlet 24 extending therefrom. The outlet 22 of the protective device 18 is affixed to the inlet 16 of the vacuum regulator 10 and, as will be later explained, provides protection to the vacuum regulator 10 from the introduction of biological liquids received from a patient during the drainage of that patient by the medical suction system.

Turning now to Fig. 2, there is a cross-sectional view of a protective device 18 of the present invention and illustrating the flow, by arrows A, of the gas through the protective device 18 as the vacuum draws gas therethrough. The housing 20 includes the outlet 22 and the inlet 24 and there is also an actuator mechanism 26 located internal of the housing 20 that has blocking surfaces 28, 30 located on opposite sides of the actuator mechanism 26 and, respectively, generally in aligned proximity to the outlet 22 and the inlet 24.

The actuator mechanism 26 can be supported within the housing 20 by a variety of means, however, as shown, there may be a plurality of light springs 32 that retain the actuator mechanism 26 in the desired position therein. In Fig. 2, the blocking surfaces 28, 30 are displaced away from the outlet 22 and the inlet 24 so that the flow of gas can pass through the protective device 18 along the path of arrows A. As will later become clear, the actuator mechanism 26 is in its contracted position in Fig. 2.

Turning now to Fig. 3, the is shown a cross-sectional view of the protective device 18 with the actuator mechanism 26 in its expanded position and, as can be seen, the blocking surfaces 28, 30 are physically occluding the outlet 22 and the inlet 24, respectively, such that flow through the protective device 18 is prevented by the occlusion of both the outlet 22 and the inlet 24 thereby effectively sealing the interior of the housing 20. Again, as will become clear, the actuator mechanism 26 is in its expanded position as shown in Fig. 3.

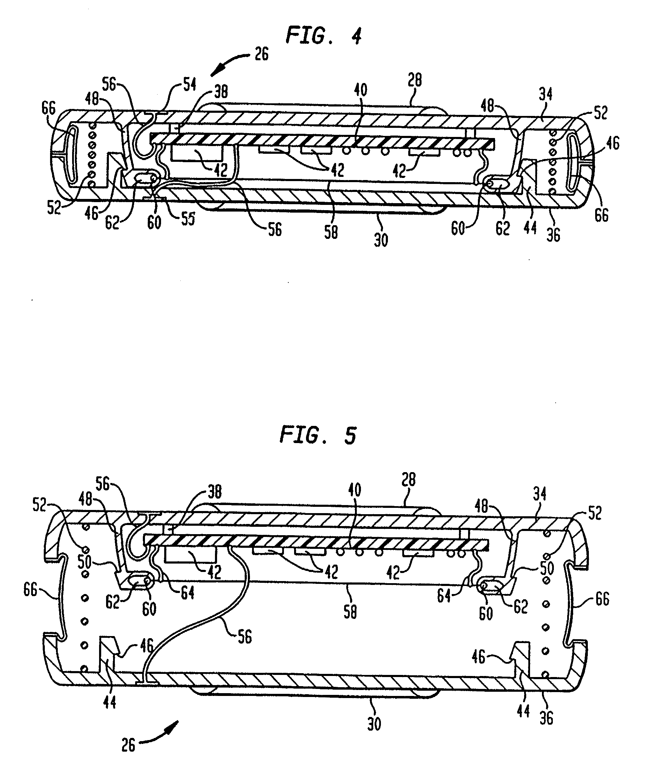

Turning now to Fig. 4, there is shown a cross-sectional view of the actuator mechanism 26 of the present invention. In this Fig., the actuator mechanism 26 is in its contracted position and comprises an upper member 34 and a lower member 36. The upper member 34 supports the blocking surface 28 and the lower member 36 supports the blocking surface 30.

A pair of standoffs 38 are formed in the upper member 34 and may be molded therein and which support a printed circuitboard 40 that has a number of electronic components 42 affixed thereto in conventional manner. There can be, of course, other means of affixing the printed circuitboard 40 within the housing 20 and it may be affixed to the lower member 36 as well. The components that are affixed to the printed circuitboard 40 will be later described.

Extending upwardly from the lower member 36, and which can be molded or affixed thereto, are a pair of fixed latch members 44. Each of the fixed latch members 44 includes a lip 46. As shown, there are two fixed latch members 44 illustrated in Fig. 4, however, there may be a greater number used with the present invention.

There are also a pair, or greater number as explained, of movable latch members 48 that depend downwardly from the upper member 34 and which also have a lip 50 formed therein. As can be seen in Fig. 4, the lips 46 of the fixed latch members 44 engage with the lips 50 of the movable latch members 48 to retain the upper member 34 latched to the lower member 36, thereby retaining the actuator mechanism 26 in its contracted position. Countering the latching of the upper and lower members 34, 36 is a bias that tends to move the actuator mechanism 26 to its expanded position, that is, the bias is provided by a pair of springs 52 that are compressed in Fig. 4 and which, therefore, push outwardly on the upper and lower members 34, 36.

A sensing electrode 54 is positioned on the external surface of the upper member 34 and a sensing electrode 55 positioned on the external surface of the lower member 36. Each of the sensing electrodes 54, 55 is connected to the electronic components 42 on the printed circuitboard 40 by means of hard wires 56. The sensing electrodes 54, 55 are shown on opposite surfaces of the actuator mechanism 26, that is, on the external surfaces of the upper and lower members 34, 36 in order to minimize the occurrences of false activations. While the sensing electrodes 54, 55 can be located adjacent to each other, there is a higher likelihood of the protective device 12 activating upon the sensing of a negligible amount of a liquid and prematurely occluding the vacuum line to the patient. With the orientation as shown, therefore, the likelihood of the false activations is less likely to occur.

There is also a shape-metal alloy wire 58 that is stretched between the movable latch members 48 and can be connected thereto by a variety of means, and one such means is the interfitting or connecting of enlarged ends 60 within openings 62 of the movable latch members 48. A further set of hard wires 64 electrically connects the electronic components 42 affixed to the printed circuitboard 40 to the shape-metal alloy wire 58. There is also located within the peripheral area of the actuator mechanism 26 and affixed to both the upper member 34 and the lower member 36, a brightly colored diaphragm 66 that is compressed within that peripheral area. As can be seen, since the diaphragm 66 is, in Fig 4, contained interiorly within the upper and lower members 34, 36, it cannot be readily seen from exterior of the actuator mechanism 26.

Due to the characteristics of a shape-metal alloy wire 58, when an electrical discharge is applied to the shape-metal alloy wire 58, there is a heating effect and thus the shape-metal alloy wire 58 contracts and pulls the movable latch members 48 inwardly so as to disengage the lip 46 of the fixed latch member 44 from the lip 50 of the movable latch member 48 thereby releasing the interengagement of the movable latch member 48 from the fixed latch member 44 to allow the spring 52 to push the upper and lower members 34, 36 apart.

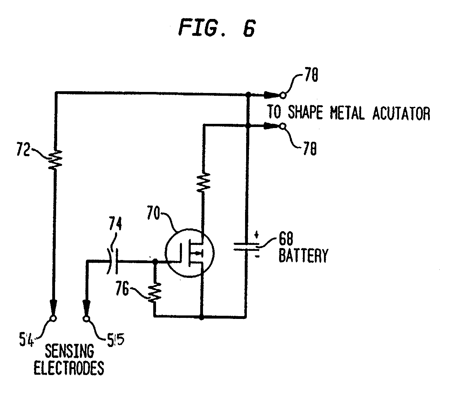

Turning now to Fig. 5, taken along with Fig. 4, there is a cross-sectional view of the actuator mechanism 26 that has been moved to its expanded position, having been pushed to that position by means of the springs 52. As can be seen, the diaphragm 66 has also expanded with the expansion of the actuator mechanism 26 to its expanded position so that the brightly colored diaphragm 66 is now readily visible through the transparent housing 20 (Figs. 2 and 3) to the attending personnel who can immediately visibly ascertain that the actuator mechanism 26 has moved to the expanded position and, as explained, the protective device 18 has effectively closed off the source of vacuum to a patient so that the necessary corrective action can be taken.

Turning now to Fig. 6, taken along with Figs. 4-5, there is a typical electrical circuit that can be used to activate the present invention and the components of Fig. 6 are those that are shown as the electronic components 42 affixed to the circuitboard 40 in Figs. 4 and 5. As stated, there are many electrical circuits that can be used and the present circuit of Fig. 6 is but one usable circuit with the invention, the object being to create a brief electrical discharge to the shape-metal alloy wire 58 when a liquid is detected between the two corrosion-resistant sensing electrodes 55.

In the circuit of Fig. 6, there is a battery 68 that is used and which powers the electrical circuit so as to provide the electrical discharge to the shape-metal alloy wire 58. The battery can be a lithium coin-type battery. The circuit includes a sensitive MOS field effect transistor 70. The sensing electrodes 54,55 are also shown and a resistor 72 limits the flow of current through the sensing electrodes 54,55 in the event of a circuit malfunction. With the circuit, when a liquid is sensed between the sensing electrodes 54,55 i.e. there is a conduction therebetween and the circuit thereby supplies an input voltage at the gate of transistor 70 through the capacitor 74. The capacitor 74 and resistor 76 form a circuit that limits the time that the transistor 70 is turned on. Thus, when the voltage is applied to the gate of transistor 70, the transistor 70 conducts and passes a current through the shape-metal alloy wire 58 connected to the terminals 78.

As such, when the sensing electrodes 54,55 sense a liquid therebetween, the transistor 70 sends a brief electrical discharge to the shape-metal alloy wire 58 so as to contract that shape-metal alloy wire 68, thereby disengaging the fixed latch members 44 from the movable latch members 48 to release the upper and lower members 34, 36 to change the actuator mechanism 26 from the Fig. 4 contracted position to the Fig. 5 expanded position, thereby occluding both the outlet 22 and the inlet 24 as described with respect to Figs 2 and 3.

Those skilled in the art will readily recognize numerous adaptations and modifications which can be made to the protective device of the present invention which will result in an improved medical vacuum system, yet all of which will fall within the scope and spirit of the present invention as defined in the following claims. Accordingly, the invention is to be limited only by the following claims and their equivalents.

## Claims

1. A protective device for a medical suction system said protective device comprising a housing having an inlet and an outlet, an actuator mechanism contained within the housing, said actuator mechanism having opposed blocking surfaces generally aligned with said inlet and said outlet and being movable between a contracted position wherein said blocking surfaces are displaced away from said inlet and said outlet and an expanded position wherein said blocking surfaces block both said inlet and said outlet, and a latch mechanism adapted to sense the presence of a liquid within said housing to move said actuator mechanism from said contracted position to said expanded position.

2. The protective device as defined in claim 1 wherein said latch mechanism includes a latch operable by a shape-metal alloy wire.

3. The protective device as defined in claim 2 wherein said latch mechanism comprises at least two movable latch members interfitted into a corresponding at least two fixed latch members, with said shape-metal alloy wire affixed between said at least two movable latch members, whereby said latch mechanism is moved to said expanded position by the contraction of said shape-metal alloy wire.

4. The protective device as defined in claim 1 wherein said actuator mechanism includes a visual indicator that is visible to alert persons that said actuator mechanism is in said expanded position.

5. A medical vacuum system for withdrawing liquids from a cavity of a patient, said system comprising:
a source of vacuum, a patient circuit for communicating the source of vacuum to a cavity of a patient, said patient circuit including a vacuum regulator,
a protective device located in the patient circuit intermediate said vacuum regulator and a patient, said protective device comprising a housing having an inlet and an outlet, an actuator mechanism contained within the housing, said actuator mechanism having opposed blocking surfaces generally aligned with said inlet and said outlet and being movable between a contracted position wherein said blocking surfaces are displaced away from said inlet and said outlet and an expanded position wherein said blocking surfaces block both said inlet and said outlet, said actuating mechanism being biased toward said expanded position and having a latch mechanism retaining said actuating mechanism in said contracted position, said latch mechanism adapted to sense the presence of a liquid within said housing to release said latch mechanism to move said actuator mechanism from said contracted position to said expanded position to terminate communication between a patient and the source of vacuum.

6. The medical vacuum system as defined in claim 5 wherein said latch mechanism includes a latch operable by a shape-metal alloy wire.

7. The medical vacuum system as defined in claim 6 wherein said latch mechanism comprises at least two movable latch members interfitted into a corresponding at least two fixed latch members, with said shape-metal alloy wire affixed between said at least two movable latch members, whereby said latch mechanism is released by the contraction of said shape-metal alloy wire.

8. A method of providing protection against liquid passing through a medical vacuum system, said method comprising the steps of:
providing a vacuum regulator adapted to be connected a source of vacuum,
providing a protective device upstream of the vacuum regulator and in fluid communication therewith, the protective device having an inlet and an outlet wherein gas normally flows through said outlet to the vacuum regulator,
sensing the presence of a liquid in said housing; and
blocking the inlet and the outlet of said protective device upon the sensing of the presence of a liquid.

9. The method as defined in claim 8 wherein the step of providing a protective device comprises providing a protective device having an actuator mechanism having an expanded position blocking the inlet and the outlet and a contracted position wherein fluid can flow between said inlet and said outlet.

10. A method as defined in claim 9 wherein said step of blocking the inlet and outlet comprises providing a shape-metal alloy wire adapted to move upon receiving an electrical discharge to cause said actuator mechanism to move from said contracted position to said expanded position.
